(19) Europäisches Patentamt
European Patent Office
Office européen des brevets

(11) EP 2 686 070 B1

(12) **EUROPEAN PATENT SPECIFICATION**

(45) Date of publication and mention of the grant of the patent:
**28.03.2018 Bulletin 2018/13**

(21) Application number: **11736465.3**

(22) Date of filing: **17.06.2011**

(51) Int Cl.:
***A61K 8/41*** *(2006.01)*
***A61K 8/88*** *(2006.01)*
***A61K 8/36*** *(2006.01)*
***A61K 8/81*** *(2006.01)*
***A61Q 1/10*** *(2006.01)*

(86) International application number:
**PCT/JP2011/064488**

(87) International publication number:
**WO 2012/124179 (20.09.2012 Gazette 2012/38)**

(54) **COSMETIC COMPOSITION FOR EYELASHES**

KOSMETISCHE ZUBEREITUNG FÜR WIMPERN

COMPOSITION COSMÉTIQUE POUR LES CILS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2011 US 201161452857 P**

(43) Date of publication of application:
**22.01.2014 Bulletin 2014/04**

(73) Proprietor: **L'Oréal**
**75008 Paris (FR)**

(72) Inventors:
- **SUGIMOTO, Naoto**
  **Kawasaki-shi**
  **Kanagawa 213-0012 (JP)**
- **PATEL, Kavita**
  **Kawasaki-shi**
  **Kanagawa 213-0012 (JP)**
- **ABE, Hiroko**
  **Kawasaki-shi**
  **Kanagawa 213-0012 (JP)**

(74) Representative: **Dossmann, Gérard**
**Casalonga & Partners**
**Bayerstrasse 71-73**
**80335 München (DE)**

(56) References cited:
**EP-A1- 0 663 202**
**EP-A1- 1 417 951**
**EP-A2- 1 345 570**
**FR-A1- 2 908 300**
**US-A1- 2004 137 021**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 686 070 B1

## Description

TECHNICAL FIELD

The present invention relates to a cosmetic composition for eyelashes such as mascaras. The present invention also relates to a cosmetic process for making up eyelashes by using the cosmetic composition.

BACKGROUND OF THE INVENTION

Many mascaras have been developed for improved cosmetic properties. For example, WO 2008/062530 discloses a cosmetic composition in the form of an O/W emulsion for eyelashes, containing soap such as triethanolamine (TEA)-stearate and 2-amino-2-methyl-1-propanol (AMP)-stearate. However, the above cosmetic composition in the form of an O/W emulsion has poor make-up properties. For example, the soap in the above cosmetic composition works as a plasticizer, which prevents lifting up of the eyelashes. Moreover, TEA and AMP bases in the O/W emulsion cause smudging around the eyes of consumers if they try to cleanse off the cosmetic composition with only warm water. Thus, there remains a need for an improved cosmetic composition which possesses not only enhanced cosmetic properties but also good performance without smudging.

DISCLOSURE OF INVENTION

An objective of the present invention is to provide a cosmetic composition for the eyelashes such as a mascara, which possesses improved cosmetic properties such as, for example, better eyelash lengthening and/or thickening and/or curling properties.

Another objective of the present invention is to provide a cosmetic composition for the eyelashes, which has a good "smudge-proof" property, which means that the cosmetic composition loaded on the lashes is neither easily smeared onto the skin surrounding the eyes nor removed by tears or water.

Another objective of the present invention is to provide a cosmetic composition for eyelashes, which has an "easy-removal" property, which means that the cosmetic composition loaded on the eyelashes can be removed as a *"tube",* or softened with warm water (about 40°C) and easily cleansed with or without a cleansing agent, preferably without a cleansing agent.

Another objective of the present invention is to provide a cosmetic composition for eyelashes which is safe and has good emulsifying properties.

The above objectives of the present invention can be achieved by a cosmetic composition for eyelashes, comprising:

- at least one anionic surfactant including 2-amino-2-methyl-1,3-propanediol in combination with a $C_{16}$-$C_{24}$ fatty acid; and
- at least one film-forming polymer present in the form of particles dispersed in an aqueous phase,

wherein
the weight ratio of the amount of 2-amino-2-methyl-1,3-propanediol in combination with the $C_{16}$-$C_{24}$ fatty acid/the amount of the film forming polymer is 0.01 to 0.3, preferably 0.05 to 0.2, and more preferably 0.1 to 0.15, including all ranges and subranges therebetween, and
the film-forming polymer is selected from vinyl (co)polymers, (meth)acrylic (copolymers, urethanes (co)polymers, and mixtures thereof.

It is preferable that the $C_{16}$-$C_{24}$ fatty acid be stearic acid.

It is preferable that the anionic surfactant be present in an amount of from 0.01 % to 10% by weight, preferably 0.1% to 5% by weight, and more preferably 0.5 to 3% by weight, relative to the total weight of the cosmetic composition, including all ranges and subranges therebetween.

The film-forming polymer may be selected from the group consisting of synthetic polymers, of the free-radical type or of the polycondensate type, and polymers of natural origin, and mixtures thereof, preferably synthetic polymers. Furthermore, the film-forming polymer may be selected from styrene-(meth)acrylic and (meth)acrylic copolymer, vinyl acetate and (meth)acrylic copolymer and mixtures thereof. The film-forming polymer may be present in an amount of dry matter from 10% to 30% by weight, preferably from 15% to 25% by weight, and more preferably 15% to 20% by weight, relative to the total weight of the cosmetic composition, including all ranges and subranges therebetween.

It is preferable that the cosmetic composition for eyelashes according to the present invention comprise at least one nonionic surfactant with an HLB of less than 8 at 25°C. The nonionic surfactant with an HLB of less than 8 at 25°C may be selected from the group consisting of saccharide esters and ethers, fatty acid esters, mixture of cyclomethicone/dimethicone copolyol, and mixtures thereof.

It is preferable that the cosmetic composition for eyelashes according to the present invention further comprise at least one nonionic surfactant with an HLB of greater than or equal to 8 at 25°C.

The nonionic surfactant with an HLB of greater than or equal to 8 at 25°C may be selected from the group consisting of oxyethylenated and/or oxypropylenated ethers of glycerol, oxyethylenated and/or oxypropylenated ethers of fatty alcohol, fatty acid esters of polyethylene glycol, fatty acid esters of oxyethylenated and/or oxypropylenated glyceryl ethers, fatty acid esters of oxyethylenated and/or oxypropylenated sorbitol ethers, dimethicone copolyol, dimethicone copolyol benzoate, copolymers of propylene oxide and of ethylene oxide, and mixtures thereof.

It is preferable that the cosmetic composition for eyelashes according to the present invention further comprise water.

It is preferable that the cosmetic composition for eyelashes according to the present invention further comprise at least one wax.

It is preferable that the cosmetic composition for eyelashes according to the present invention further comprise at least one fiber.

The present invention also relates to a cosmetic process for making-up eyelashes, comprising the steps of:

- loading an applicator with the cosmetic composition according to the present invention; and
- applying said cosmetic composition onto the eyelashes.

DETAILED DESCRIPTION OF THE INVENTION

The inventors performed diligent research and found that a cosmetic composition for eyelashes, which comprises at least one anionic surfactant including 2-amino-2-methyl-1,3-propanediol in combination with a $C_{16}$-$C_{24}$ fatty acid, and at least one film-forming polymer present in the form of particles dispersed in an aqueous phase, wherein the weight ratio of the amount of 2-amino-2-methyl-1,3-propanediol in combination with the $C_{16}$-$C_{24}$ fatty acid/the amount of the film forming polymer is 0.01 to 0.3, can improve the cosmetic performance, such as curling, lengthening, and thickening of the eyelashes, and possesses a good "smudge-proof" property or "easy-removal" property. In addition, the cosmetic composition for eyelashes are safe and have good emulsifying properties.

Thus, the cosmetic composition according to the present invention is characterized by comprising:

- at least one anionic surfactant including 2-amino-2-methyl-1,3-propanediol in combination with a $C_{16}$-$C_{24}$ fatty acid; and
- at least one film-forming polymer present in the form of particles dispersed in an aqueous phase,

wherein

the weight ratio of the amount of the 2-amino-2-methyl-1,3-propanediol in combination with the $C_{16}$-$C_{24}$ fatty acid/the amount of the film forming polymer is 0.01 to 0.3, preferably 0.05 to 0.2, and more preferably 0.1 to 0.15, including all ranges and subranges therebetween, and

the film-forming polymer is selected from vinyl (copolymers (meth)acrylic (co)polymers, urethanes (copolymers, and mixtures thereof.

Hereinafter, the cosmetic composition according to the present invention will be explained in a more detailed manner. Unless otherwise specified, the weight ratios described herein are expressed in dry weight.

Anionic surfactants

The cosmetic composition according to the present invention comprises, in combination with 2-amino-2-methyl-1,3-propanediol, at least one $C_{16}$-$C_{24}$ fatty acid. Two or more fatty acids may be used.

The term "fatty acid" here means a carboxylic acid with a long aliphatic carbon chain. It is preferable that the fatty acid be selected from any saturated or unsaturated, linear or branched fatty acids.

Mention may be made among these of palmitic acid, margaric acid, stearic acid, nonadecylic acid, arachidic acid, heneicosylic acid, behenic acid, tricosylic acid, lignoceric acid, and mixtures thereof.

2-amino-2-methyl-1,3-propanediol stearate is most particularly suitable for the present invention. Such a surfactant is generally obtained by simply mixing stearic acid with 2-amino-2-methyl-1,3-propanediol.

According to the present invention, other than those fatty acid salts derived from the above-mentioned fatty acids and amines, the cosmetic composition may further comprise at least one anionic surfactant. As non-limiting examples of such anionic surfactant, mention may be made more particularly of:

- polyoxyethylenated fatty acid salts, especially those derived from amines or alkali metal salts, and mixtures thereof;

- phosphoric esters and salts thereof, such as "DEAoleth-10 phosphate" ("Crodafos N 10N" from the company Croda);
- sulfosuccinates such as "Disodium PEG-5 citrate lauryl sulfosuccinate" and "Disodium ricinoleamido MEA sulfosuccinte";
- alkyl ether sulfates, such as sodium lauryl ether sulfate;
- isethionates;
- acylglutamate such as "Disodium hydrogenated tallow glutamate" ("Amisoft HS-21 R" sold by the company Ajinomoto); and
- mixtures thereof.

The anionic surfactant mat be present in the cosmetic composition for eyelashes according to the present invention in an amount of from 0.01% to 10% by weight, preferably 0.1% to 5% by weight, and more preferably 0.5 to 3% by weight, relative to the total weight of the cosmetic composition, including all ranges and subranges therebetween.

Film-forming polymers

The cosmetic composition according to the present invention comprises at least one film-forming polymer present in the form of particles dispersed in an aqueous phase, which is generally known as a latex.

In the present patent application, the term "film-forming polymer" means a polymer capable of, by itself or in the presence of an auxiliary film-forming agent, forming a continuous film that adheres to a support and especially to keratin materials, for instance the eyelashes.

The film-forming polymer may be present in the cosmetic composition according to the present invention in an amount of dry matter from 10% to 30% by weight, preferably from 15% to 25% by weight, and more preferably from 15% to 20% by weight, relative to the total weight of the cosmetic composition, including all ranges and subranges therebetween.

Among the film-forming polymers that may be used in the cosmetic composition of the present invention, mention may be made of synthetic polymers, of the free-radical type or of the polycondensate type, polymers of natural origin, and mixtures thereof.

The film-forming polymer according to the present invention is selected from vinyl (co)polymers, (meth)acrylic (co)polymers, urethanes (co)polymers, and mixtures thereof. Advantageously, the film-forming polymer is selected from a styrene-(meth)acrylic and (meth)acrylic copolymer, a vinyl acetate and (meth)acrylic copolymer, and mixtures thereof.

The film-forming polymers of the free-radical type may be chosen, for example, from vinyl polymers or copolymers, such as acrylic polymers.

The vinyl film-forming polymers can result from the polymerization of monomers comprising at least one ethylenic unsaturation and at least one acidic group and/or esters of these acidic monomers and/or amides of these acidic monomers.

Monomers comprising at least one acid group which may be used include, for example, $\alpha,\beta$-ethylenic unsaturated carboxylic acids such as acrylic acid, methacrylic acid, crotonic acid, maleic acid or itaconic acid. (Meth)acrylic acid and crotonic acid are, for example, used. In one embodiment, (meth)acrylic acid is used.

The esters of acidic monomers are chosen, for example, from (meth)acrylic acid esters (also known as (meth)acrylates), such as (meth)acrylates of an alkyl, for example, a $C_1$-$C_{30}$ alkyl, such as a $C_1$-$C_{20}$ alkyl, (meth)acrylates of an aryl, such as a $C_6$-$C_{10}$ aryl, and (meth)acrylates of a hydroxyalkyl, such as a $C_2$-$C_6$ hydroxyalkyl.

Among the alkyl (meth)acrylates that may be mentioned, examples include methyl methacrylate, ethyl methacrylate, butyl methacrylate, isobutyl methacrylate, 2-ethylhexyl methacrylate, lauryl methacrylate and cyclohexyl methacrylate.

Among the hydroxyalkyl (meth)acrylates that may be mentioned, examples include hydroxyethyl acrylate, 2-hydroxypropyl acrylate, hydroxyethyl methacrylate and 2-hydroxypropyl methacrylate.

Among the aryl (meth)acrylates that may be mentioned, examples include benzyl acrylate and phenyl acrylate.

The (meth)acrylic acid esters that may be used are, for example, alkyl (meth)acrylates.

As disclosed herein, the alkyl group of the esters may be either fluorinated or perfluorinated, i.e., some or all of the hydrogen atoms of the alkyl group are substituted with fluorine atoms.

Examples of amides of the acid monomers that may be mentioned include (meth)acrylamides, such as N-alkyl(meth)acrylamides, for example, of a $C_2$-$C_{12}$ alkyl. Among the N-alkyl(meth)acrylamides that may be mentioned, examples include N-ethylacrylamide, N-t-butylacrylamide, N-t-octylacrylamide and N-undecylacrylamide.

The vinyl film-forming polymers may also result from the homopolymerization or copolymerization of monomers chosen from vinyl esters and styrene monomers. For example, these monomers may be polymerized with acid monomers and/or esters thereof and/or amides thereof, such as those mentioned above.

Examples of vinyl esters that may be mentioned include vinyl acetate, vinyl neodecanoate, vinyl pivalate, vinyl benzoate and vinyl t-butylbenzoate. Styrene monomers that may be mentioned include styrene and $\alpha$-methylstyrene.

Among the film-forming polycondensates that may be mentioned, examples include polyurethanes.

The polyurethanes may be chosen from anionic, cationic, nonionic or amphoteric polyurethanes, polyurethane-acrylics, polyurethane-polyvinylpyrrolidones, polyester-polyurethanes, polyether-polyurethanes, polyureas and polyurea-polyurethanes, and mixtures thereof.

According to one embodiment of the cosmetic composition according to the present invention, the film-forming polymer may be a liposoluble polymer.

Examples of the liposoluble polymer that may be mentioned include copolymers of a vinyl ester (wherein the vinyl group is directly linked to the oxygen atom of the ester group and the vinyl ester comprises a radical chosen from saturated, linear or branched hydrocarbon-based radicals of 1 to 19 carbon atoms, linked to the carbonyl of the ester group) and of at least one other monomer, which may be a vinyl ester (different from the vinyl ester already present), an $\alpha$-olefin (comprising from 8 to 28 carbon atoms), an alkyl vinyl ether (the alkyl group of which comprises from 2 to 18 carbon atoms) or an allylic or methallylic ester (comprising a radical chosen from saturated, linear or branched hydrocarbon-based radicals of 1 to 19 carbon atoms, linked to the carbonyl of the ester group).

These copolymers may be crosslinked using crosslinking agents that may be either of the vinylic type or of the allylic or methallylic type, such as tetraallyloxyethane, divinylbenzene, divinyl octanedioate, divinyl dodecanedioate and divinyl octadecanedioate.

Examples of these copolymers which may be mentioned include the following copolymers: vinyl acetate/allyl stearate, vinyl acetate/vinyl laurate, vinyl acetate/vinyl stearate, vinyl acetate/octadecene, vinyl acetate/octadecyl vinyl ether, vinyl propionate/allyl laurate, vinyl propionate/vinyl laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/vinyl laurate, allyl 2,2-dimethylpentanoate/vinyl laurate, vinyl dimethylpropionate/vinyl stearate, allyl dimethylpropionate/vinyl stearate, vinyl propionate/vinyl stearate, crosslinked with 0.2% divinylbenzene, vinyl dimethylpropionate/vinyl laurate, crosslinked with 0.2% divinylbenzene, vinyl acetate/octadecyl vinyl ether, crosslinked with 0.2% tetraallyloxyethane, vinyl acetate/allyl stearate, crosslinked with 0.2% divinylbenzene, vinyl acetate/1-octadecene, crosslinked with 0.2% divinylbenzene, and allyl propionate/allyl stearate, crosslinked with 0.2% divinylbenzene.

Examples of the liposoluble film-forming polymers which may also be mentioned include liposoluble copolymers, such as those resulting from the copolymerization of vinyl esters comprising from 9 to 22 carbon atoms or of alkyl acrylates or methacrylates, wherein the alkyl radicals comprise from 10 to 20 carbon atoms.

Such liposoluble copolymers may be chosen, for example, from polyvinyl stearate, polyvinyl stearate crosslinked with the aid of divinylbenzene, of diallyl ether or of diallyl phthalate copolymers, polystearyl (meth)acrylate, polyvinyl laurate and polylauryl (meth)acrylate copolymers, it being possible for these poly(meth)acrylates to be crosslinked with the aid of ethylene glycol dimethacrylate or tetraethylene glycol dimethacrylate.

The liposoluble copolymers defined above are known and are described, for example, in French patent application FR-A-2 232 303; they may have a weight-average molecular weight ranging, for example, from 2,000 to 500,000 such as from 4,000 to 200,000.

Among the liposoluble film-forming polymers which may be used herein, mention may also be made, for example, of copolymers of vinylpyrrolidone (VP) such as the copolymers of VP/vinyl acetate, VP/ethyl methacrylate, VP/ethyl methacrylate/ methacrylic acid, VP/styrene or VP/acrylic acid/lauryl methacrylate.

In some embodiments, the film-forming polymer has a Tg (glass transition temperature) value of less than 50°C.

Aqueous dispersions of film-forming polymers which may be used are the acrylic dispersions sold under the names "Neocryl XK-90®", "Neocryl A-1070®", "Neocryl A-1090®", "Neocryl BT-62®", "Neocryl A-1079®" and "Neocryl A-523®" by the company Avecia-Neoresins, "Dow Latex 432®" by the company Dow Chemical, "Daitosol 5000 AD®" or "Daitosol 5000 SJ" by the company Daito Kasey Kogyo; "Syntran 5760" by the company Interpolymer or the aqueous dispersions of polyurethane sold under the names "Neorez R-981®" and "Neorez R-974®" by the company Avecia-Neoresins, "Avalure UR-405®", "Avalure UR-410®", "Avalure UR-425®", "Avalure UR-450®", "Sancure 875®", "Sancure 861®", "Sancure 878®" and "Sancure 2060®" by the company Goodrich, "Impranil 85®" by the company Bayer and "Aquamere H-1511®" by the company Hydromer; vinyl dispersions, for instance "Mexomer PAM" and also acrylic dispersions in isododecane, for instance "Mexomer PAP" by the company Chimex.

<u>Nonionic surfactant with an HLB of less than 8</u>

The cosmetic composition according to the present invention may comprise at least one nonionic surfactant with an HLB of less than 8 at 25°C.

As non-limiting illustrations of these nonionic surfactants with an HLB of less than 8 at 25°C, mention may be made more particularly of:

- saccharide esters and ethers, such as sucrose stearate, sucrose cocoate, sorbitan stearate, and mixtures thereof, for instance "Arlatone 2121" sold by the company ICI;

- fatty acid esters (especially of a $C_8$-$C_{24}$ acid and preferably a $C_{16}$-$C_{22}$ acid) of polyol, especially of glycerol or of sorbitol, for instance, glyceryl stearate such as the product sold under the name "Tegin M" by the company Goldschmidt; glyceryl laurate such as the product sold under the name "Imwitor 312" by the company Hüls; polyglyceryl-2 stearate, sorbitan tristearate or glyceryl ricinoleate; and
- a mixture of cyclomethicone/dimethicone copolyol sold under the name "Q2-3225C" by the company Dow Corning.

The amount of nonionic surfactant is generally adjusted so as to obtain a composition having the parameters as defined above. The determination of this amount falls within the competence of a person skilled in the art.

As non-limiting examples of the scope of the invention, the amount of the nonionic surfactant(s) with an HLB of less than 8 at 25°C may range from 0.01% to 40% by weight, preferably 0.1% to 25% by weight, more preferably 0.2% to 15% by weight, and even more preferably 0.4% to 10% by weight, relative to the total weight of the composition, including all ranges and subranges therebetween.

<u>Nonionic surfactant with an HLB of greater than or equal to 8</u>

According to the present invention, the cosmetic composition may further comprise at least one nonionic surfactant with an HLB of greater than equal to 8 at 25°C.

As non-limiting illustrations of nonionic surfactants with an HLB of greater than equal to 8 at 25°C that may be used, alone or as a mixture, in the makeup compositions according to the invention, mention may be made especially of:

- oxyethylenated and/or oxypropylenated ethers (which may comprise from 1 to 150 oxyethylene and/or oxypropylene groups) of glycerol;
- oxyethylenated and/or oxypropylenated ethers (which may comprise from 1 to 150 oxyethylene and/or oxypropylene groups) of fatty alcohols (especially of a $C_8$-$C_{24}$ alcohol and preferably a $C_{12}$-$C_{18}$ alcohol), such as oxyethylenated cetearyl alcohol ether containing 30 oxyethylene groups (CTFA name "Ceteareth-30"), oxyethylenated stearyl alcohol ether containing 20 oxyethylene groups (CTFA name "Steareth-20"), and the oxyethylenated ether of the mixture of $C_{12}$-$C_{15}$ fatty alcohols comprising 7 oxyethylene groups (CTFA name "$C_{12-15}$ Pareth-7" sold under the name "Neodol 25-7®" by Shell Chemicals);
- fatty acid esters (especially of a $C_8$-$C_{24}$ acid and preferably a $C_{16}$-$C_{22}$ acid) of polyethylene glycol (which may comprise from 1 to 150 ethylene glycol units), such as PEG-50 stearate and PEG-40 monostearate sold under the name "Myrj 52P" by the company ICI Uniquema;
- fatty acid esters (especially of a $C_8$-$C_{24}$ acid and preferably a $C_{16}$-$C_{22}$ acid) of oxyethylenated and/or oxypropylenated glyceryl ethers (which may comprise from 1 to 150 oxyethylene and/or oxypropylene groups), for instance PEG-200 glyceryl monostearate sold under the name "Simulsol 200 TM" by the company SEPPIC; glyceryl stearate polyethoxylated with 30 ethylene oxide groups, for instance the product "Tagat S" sold under the company Goldschmidt; glyceryl oleate polyethoxylated with 30 ethylene oxide groups, for instance the product "Tagat O" sold by the company Goldschmidt; glyceryl cocoate polyethoxylated with 30 ethylene oxide groups, for instance the product "Varionic LI 13" sold by the company Sherex; glyceryl isostearate polyethoxylated with 30 ethylene oxide groups, for instance the product "Tagat L" sold by the company Goldschmidt; and glyceryl laurate polyethoxylated with 30 ethylene oxide groups, for instance the product "Tagat I" form the company Goldschmidt;
- fatty acid esters (especially of a $C_8$-$C_{24}$ acid and preferably a $C_{16}$-$C_{22}$ acid) of oxyethylenated and/or oxypropylenated sorbitol ethers (which may comprise from 1 to 150 oxyethylene and/or oxypropylene groups), for instance polysorbate 60 sold under the name "Tween 60" by the company Uniqema;
- dimethicone copolyol, such as the product sold under the name "Q2-5220" by the company Dow Corning;
- dimethicone copolyol benzoate (Finsolv SLB 101 and 201 by the company Finetex);
- copolymers of propylene oxide and of ethylene oxide, also known as EO/PO polycondensates; and
- mixtures thereof.

The EO/PO polycondensates are more particularly copolymers consisting of polyethylene glycol and polypropylene glycol blocks, for instance polyethylene glycol/polypropylene glycol/polyethylene glycol triblock polycondensates. These triblock polycondensates have, for example, the following chemical structure:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

in which a ranges from 2 to 120 and b ranges from 1 to 100.

The EO/PO polycondensate preferably has a weight-average molecular weight ranging from 1,000 to 15,000 and better still ranging from 2,000 to 13,000. Advantageously, said EO/PO polycondensate has a cloud point, at 10 g/l in distilled water, of greater than or equal to 20°C, and preferably greater than or equal to 60°C. The cloud point is

measured according to ISO standard 1065. As EO/PO polycondensates that may be used according to the invention, mention may be made of the polyethylene glycol/polypropylene glycol/polyethylene glycol triblock polycondensates sold under the name "Synperonic", for instance "Synperonic PE/L44" and "Synperonic PE/F127", by the company ICI, and mixtures thereof.

As non-limiting examples of the scope of the invention, the amount of the nonionic surfactant(s) with an HLB of greater than equal to 8 at 25°C may range from 0.01% to 40% by weight, preferably 0.1% to 25% by weight, more preferably 0.2% to 15% by weight, and even more preferably 0.4% to 10% by weight, relative to the total weight of the composition, including all ranges and subranges therebetween.

Cosmetically acceptable medium

The cosmetic composition according to the present invention may comprise a cosmetically acceptable medium such as an aqueous medium. The aqueous medium in the cosmetic composition according to the present invention may comprise water.

The amount of water may be present in an amount ranging from 15% to 50% by weight, preferably from 20% to 40% by weight, and more preferably from 25% to 35% by weight, relative to the total weight of the composition. The amount of water mentioned above includes the amount of water present in the aqueous phase where at least one film-forming polymer is present in the form of dispersed particles.

The aqueous medium may further comprise at least one organic solvent. The organic solvent is preferably water-miscible. As the organic solvent, there may be mentioned, for example, $C_1$-$C_4$ alkanols, such as ethanol and isopropanol; glycerol; glycols and glycol ethers such as 2-butoxyethanol, propylene glycol, monomethyl ether of propylene glycol, monoethyl ether and monomethyl ether of diethylene glycol; and aromatic alcohols such as benzyl alcohol and phenoxyethanol; analogous products; and mixtures thereof.

The organic solvents may be present in an amount ranging from 1% to 40 % by weight, preferably from 1% to 30% by weight, and more preferably from 5% to 20% by weight, relative to the total weight of the composition, including all ranges and subranges therebetween.

The cosmetic compositions disclosed herein may also comprise ingredients commonly used in the field of makeup for keratin fibers.

For example, the cosmetic composition disclosed herein may comprise at least one oil.

The at least one oil may be chosen from volatile oils and non-volatile oils, and mixtures thereof. In one embodiment, the presently disclosed composition comprises at least one volatile oil.

As used herein, the term "volatile oil" means an oil that is capable of evaporating on contact with the skin or a keratin fiber in less than one hour, at room temperature and atmospheric pressure. The at least one volatile organic solvent and the at least one volatile oil disclosed herein are volatile organic solvents and cosmetic oils that are liquid at room temperature, with a non-zero vapor pressure at room temperature and atmospheric pressure, ranging, for example, from 0.13 Pa to 40,000 Pa ($10^{-3}$ to 300 mmHg), such as from 1.3 Pa to 13,000 Pa (0.01 to 100 mmHg), and further such as from 1.3 Pa to 1,300 Pa(0.01 to 10 mmHg). The term "non-volatile oil" means an oil that remains on the skin or the keratin fiber at room temperature and atmospheric pressure for at least several hours and that has, for example, a vapor pressure of less than $10^{-3}$ mmHg (0.13 Pa).

These oils may be hydrocarbon-based oils, silicone oils or fluoro oils, or mixtures thereof.

As used herein, the term "hydrocarbon-based oil" means an oil mainly comprising hydrogen and carbon atoms and optionally oxygen, nitrogen, sulfur and/or phosphorus atoms. The volatile hydrocarbon-based oils may be chosen, for example, from hydrocarbon-based oils comprising from 8 to 16 carbon atoms, such as branched $C_8$-$C_{16}$ alkanes, for instance $C_8$-$C_{16}$ isoalkanes of petroleum origin (also known as isoparaffins), such as isododecane (also known as 2,2,4,4,6-pentamethylheptane), isodecane and isohexadecane, for example, the oils sold under the trade names Isopar or Permetyl, branched $C_8$-$C_{16}$ esters and isohexyl neopentanoate, and mixtures thereof. Other volatile hydrocarbon-based oils, for instance petroleum distillates, such as those sold under the name Shell Solt by the company Shell, may also be used. The volatile solvent is chosen, for example, from volatile hydrocarbon-based oils comprising from 8 to 16 carbon atoms, and mixtures thereof.

The volatile oils that may also be used include, for example, volatile silicones, for instance volatile linear or cyclic silicone oils, such as those with a viscosity ≦ 8 centistokes ($8\times10^{-5} m^2/s$) and, for example, comprising from 2 to 7 silicon atoms, these silicones optionally comprising at least one group chosen from alkyl and alkoxy groups comprising from 1 to 10 carbon atoms. Among the volatile silicone oils that may be used herein, mention may be made, for example, of octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane and dodecamethyl pentasiloxane, and mixtures thereof.

Volatile fluorinated solvents such as nonafluoromethoxybutane or perfluoromethylcyclopentane may, for example, also be used.

The volatile oil may be present in the cosmetic composition as disclosed herein in an amount ranging, for example, from 0.1% to 60% by weight, such as from 0.1 % to 30% by weight, relative to the total weight of the composition, including all ranges and subranges therebetween.

The cosmetic composition may also comprise at least one non-volatile oil chosen, for example, from non-volatile hydrocarbon-based oils, silicone oils, and fluoro oils.

Non-volatile hydrocarbon-based oils that may be mentioned include, for example:

- hydrocarbon-based oils of plant origin, such as triglycerides comprising fatty acid esters of glycerol, the fatty acids of which may have varied chain lengths from $C_4$ to $C_{24}$, these chains possibly being linear or branched, and saturated or unsaturated; these oils are chosen, for example, from wheatgerm oil, sunflower oil, grapeseed oil, sesame seed oil, maize oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel,
- synthetic ethers comprising from 10 to 40 carbon atoms;
- linear or branched hydrocarbons of mineral or synthetic origin, such as petroleum jelly, polydecenes, hydrogenated polyisobutene such as parleam, and squalane, and mixtures thereof;
- synthetic esters such as oils of formula $R_1COOR_2$ wherein $R_1$ is chosen from linear and branched fatty acid residues comprising from 1 to 40 carbon atoms and $R_2$ is chosen from branched hydrocarbon-based chains comprising from 1 to 40 carbon atoms, provided that the number of carbon atoms is $R_1+R_2 \geqq 10$, such as, purcellin oil (cetostearyl octanoate), isopropyl myristate, isopropyl palmitate, $C_{12}$-$C_{15}$ alkyl benzoate, hexyl laurate, diisopropyl adipate, isononyl isononanoate, 2-ethylhexyl palmitate, isostearyl isostearate, alkyl or polyalkyl octanoates, decanoates or ricinoleates such as propylene glycol dioctanoate; hydroxylated esters such as isostearyl lactate and diisostearyl malate; and pentaerythritol esters;
- fatty alcohols that are liquid at room temperature, comprising a branched and/or unsaturated carbon-based chain comprising from 12 to 26 carbon atoms, for instance octyldodecanol, isostearyl alcohol, oleyl alcohol, 2-hexyldecanol, 2-butyloctanol and 2-undecylpentadecanol;
- higher fatty acids such as oleic acid, linoleic acid and linolenic acid; and
- mixtures thereof.

The non-volatile silicone oils that may be used in the cosmetic composition as disclosed herein may, for example, be non-volatile polydimethylsiloxanes (PDMSs), polydimethylsiloxanes comprising alkyl or alkoxy groups, that are pendent and/or at the end of a silicone chain, wherein the alkyl or alkoxy groups each comprise from 2 to 24 carbon atoms, phenyl silicones, for instance phenyl trimethicones, phenyl dimethicones, phenyltrimethylsiloxydiphenylsiloxanes, diphenyl dimethicones, diphenylmethyldiphenyltrisiloxanes and 2-phenylethyl trimethylsiloxysilicates.

The fluoro oils that may be used herein include, for example, fluorosilicone oils, fluoropolyethers or fluorosilicones, as described in document EP-A-847 752.

The non-volatile oils may be present in the cosmetic composition as disclosed herein in an amount ranging, for example, from 0.1 % to 20% by weight, such as from 0.1 % to 12% by weight, relative to the total weight of the composition, including all ranges and subranges therebetween.

Waxes

The cosmetic composition as disclosed herein may comprise a wax or a mixture of waxes.

The wax used herein is generally a lipophilic compound that is solid at room temperature (25°C), with a solid/liquid reversible change of state, having a melting point of greater than or equal to 30°C, which may be up to 120°C.

By bringing the wax into a liquid state (melting), it is possible to make it miscible with oils and to form a microscopically uniform mixture, but on cooling the mixture to room temperature, recrystallization of the wax in the oils of the mixture is obtained. For example, the waxes that may be used herein may have a melting point of greater than 45°C, such as greater than or equal to 50°C and further such as greater than or equal to 55°C. The melting point of the wax may be measured using a differential scanning calorimeter (DSC), for example, the calorimeter sold under the name DSC 30 by the company Mettler. The measuring protocol is as follows.

A sample of 15 mg of product placed in a crucible is subjected to a first temperature rise ranging from 0°C to 120°C, at a heating rate of 10°C/minute, it is then cooled from 120°C to 0°C at a cooling rate of 10°C/minute and is finally subjected to a second temperature increase ranging from 0°C to 120°C at a heating rate of 5°C/minute. During the second temperature increase, the variation of the difference in power absorbed by the empty crucible and by the crucible containing the sample of product is measured as a function of the temperature. The melting point of the compound

is the temperature value corresponding to the top of the peak of the curve representing the variation in the difference in absorbed power as a function of the temperature.

The waxes that may be used in the cosmetic compositions disclosed herein are chosen from waxes that are solid and rigid at room temperature, of animal, plant, mineral or synthetic origin, and mixtures thereof.

The wax may also have a hardness ranging, for example, from 0.05 MPa to 30 MPa such as from 6 MPa to 15 MPa. The hardness is determined by measuring the compressive strength, measured at 20°C using the texturometer sold under the name TA-TX2i by the company Rheo, equipped with a stainless-steel cylinder 2 mm in diameter travelling at a measuring speed of 0.1 mm/s, and penetrating into the wax to a penetration depth of 0.3 mm. The measuring protocol is as follows.

The wax is melted at a temperature equal to the melting point of the wax +20°C. The molten wax is cast in a container 30 mm in diameter and 20 mm deep. The wax is recrystallized at room temperature (25°C) over 24 hours and is then stored for at least 1 hour at 20°C before performing the hardness measurement. The value of the hardness is the maximum compressive strength measured divided by the area of the texturometer cylinder in contact with the wax.

Hydrocarbon-based waxes such as beeswax, lanolin wax and Chinese insect waxes; rice wax, carnauba wax, candelilla wax, ouricury wax, esparto grass wax, cork fibre wax, sugar cane wax, Japan wax and sumach wax; montan wax, microcrystalline waxes, paraffins and ozokerite; polyethylene waxes, the waxes obtained by Fisher-Tropsch synthesis and waxy copolymers, and also esters thereof, may, for example, be used.

The waxes obtained by catalytic hydrogenation of animal or plant oils comprising linear or branched $C_8$-$C_{32}$ fatty chains, may, for example, also be used.

Among these oils, mention may be made, for example, of hydrogenated jojoba oil, isomerized jojoba oil such as the trans-isomerized partially hydrogenated jojoba oil manufactured or sold by the company Desert Whale under the trade name "Iso-Jojoba-50®", hydrogenated sunflower oil, hydrogenated castor oil, hydrogenated coconut oil and hydrogenated lanolin oil, bis(1,1,1-trimethylolpropane) tetrastearate sold under the name "Hest 2T-4S" by the company Heterene, and bis(1,1,1-trimethylolpropa- ne) tetrabehenate sold under the name "Hest 2T-4B" by the company Heterene.

Silicone waxes and fluoro waxes may, for example, also be used.

Further, for example, the wax obtained by hydrogenation of olive oil esterified with stearyl alcohol, sold under the name "Phytowax Olive 18 L 57", or the waxes obtained by hydrogenation of castor oil esterified with cetyl alcohol, sold under the name "Phytowax Ricin 16L64 and 22L73" by the company Sophim, may also be used. Such waxes are described in French patent application FR-A-2 792 190.

In one embodiment, the cosmetic composition as disclosed herein may comprise at least one "tacky" wax, i.e., a wax with a tack of greater than or equal to 0.7 N.s and a hardness of less than or equal to 3.5 MPa. Using a tacky wax may, for example, make it possible to obtain a cosmetic composition that applies easily to keratin fibers, attaches well to the keratin fibers and leads to the formation of a smooth, uniform and thickening makeup result. The tacky wax used may, for example, have a tack ranging from 0.7 N.s to 30 N.s, such as greater than or equal to 1 N.s, for example, from 1 N.s to 20 N.s, further such as greater than or equal to 2 N.s, for example, from 2 N.s to 10 N.s and further, for example, from 2 N.s to 5 N.s. The tack of the wax is determined by measuring the change in force (compression force or stretching force) as a function of time, at 20°C, using the texturometer sold under the name "TA-TX2i®" by the company Rheo, equipped with a conical acrylic polymer spindle forming an angle of 45°. The measuring protocol is as follows.

The wax is melted at a temperature equal to the melting point of the wax +10° C. The molten wax is poured into a container 25 mm in diameter and 20 mm deep. The wax is recrystallized at room temperature (25°C) for 24 hours such that the surface of the wax is flat and smooth, and the wax is then stored for at least 1 hour at 20°C before measuring the tack.

The texturometer spindle is displaced at a speed of 0.5 mm/s then penetrates the wax to a penetration depth of 2 mm. When the spindle has penetrated the wax to a depth of 2 mm, the spindle is held still for 1 second (corresponding to the relaxation time) and is then withdrawn at a speed of 0.5 mm/s.

During the relaxation time, the force (compression force) decreases greatly until it becomes zero, and then, during the withdrawal of the spindle, the force (stretching force) becomes negative and then rises again to the value 0. The tack corresponds to the integral of the curve of the force as a function of time for the part of the curve corresponding to negative values of the force (stretching force). The tack value is expressed in N.s.

The tacky wax that may be used generally has, for example, a hardness of less than or equal to 3.5 MPa, such as from 0.01 MPa to 3.5 MPa, further such as from 0.05 MPa to 3 MPa, and even further such as from 0.1 MPa to 2.5 MPa.

The hardness is measured according to the protocol described above.

Tacky waxes that may be used include a $C_{20}$-$C_{40}$ alkyl (hydroxystearyloxy)stearate (wherein the alkyl group comprises from 20 to 40 carbon atoms), alone or as a mixture, such as a $C_{20}$-$C_{40}$ alkyl 12-(12'-hydroxystearyloxy)stearate, of formula shown below:

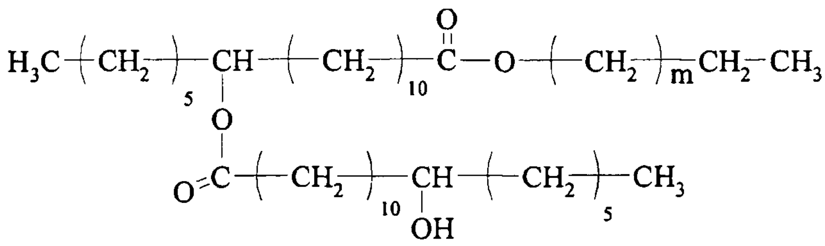

wherein m is an integer ranging from 18 to 38, or a mixture of compounds thereof.

Such a wax is, for example, sold under the names "Kester Wax K 82 P®" and "Kester Wax K 80 P®" by the company Koster Keenan.

The waxes mentioned above generally have, for example, a starting melting point of less than 45°C.

The cosmetic composition as disclosed herein may comprise a total wax content ranging, for example, from 1% to 50% by weight, such as from 5% to 40% by weight, further such as from 10% to 35% by weight, and even further such as from 10% to 30% by weight, relative to the total weight of the composition, including all ranges and subranges therebetween.

As disclosed herein, it is also possible to use waxes supplied in the form of small particles having a size, expressed as the mean "effective" volume diameter D[4,3], ranging, for example, from 0.5 to 30 micrometers, such as from 1 to 20 micrometers, further such as from 5 to 10 micrometers in size, which are used herein as "microwaxes".

The particle sizes may be measured by various techniques; mention may be made, for example, of light-scattering techniques (dynamic or static), Coulter counter methods, sedimentation rate measurements (related to the size via Stoke’s lax) and microscopy. These techniques make it possible to measure a particle diameter and, for some of them, a particle size distribution.

The sizes and size distributions of the particles in the cosmetic compositions as disclosed herein are, for example, measured by static light scattering using a commercial granulometer such as the MasterSizer 2000 from Malvern. The data are processed on the basis of the Mie scattering theory. This theory, which is exact for isotropic particles, makes it possible to determine an "effective" particle diameter in the case of non-spherical particles. This theory is described, for example, in the publication by Van de Hulst, H. C., "Light Scattering by Small Particles", Chapters 9 and 10, Wiley, New York, 1957.

The microwax used herein is characterized by its mean "effective" diameter by volume D[4,3], defined in the following manner:

$$D[4,3] = \frac{\sum_i V_i \cdot d_i}{\sum_i V_i}$$

wherein $V_i$ is the volume of the particles with an effective diameter $d_i$. This parameter D[4,3] is described, for example, in the technical documentation of the granulometer.

The measurements are performed at 25°C on a dilute particle dispersion, obtained from the microwax in the following manner: 1) dilution by a factor of 100 with water, 2) homogenization of the solution, 3) standing of the solution for 18 hours, 4) recovery of the whitish uniform supernatant.

The "effective" diameter is obtained by taking a refractive index of 1.33 for water and a mean refractive index of 1.42 for the particles.

Among the microwaxes that may be used in the cosmetic compositions as disclosed herein, mention may be made, for example, of carnauba microwaxes, such as the product sold under the name "MicroCare 350®" by the company Micro Powders, synthetic microwaxes, such as the product sold under the name "MicroEase 114S®" by the company Micro Powders, microwaxes comprising a mixture of carnauba wax and polyethylene wax, such as the products sold under the name "Micro Care 300®" and "Micro Care 310®" by the company Micro Powders, microwaxes comprising a mixture of carnauba wax and synthetic wax, such as the product sold under the name "Micro Powders 325®" by the company Micro Powders, polyethylene microwaxes, such as the products sold under the names "Micropoly 200®", "Micropoly 220®", "Micropoly 220L®", and "Micropoly 250S®" by the company Micro Powders, and polytetrafluoroethylene micropowders, such as the products sold under the names "Microslip 519®" and "Microslip 519L®" by the company Micro Powders.

The waxes (including the tacky wax) may be present in the form of an aqueous microdispersion of wax. The term "aqueous microdispersion of wax" means an aqueous dispersion of wax particles in which the size of the wax

particles is less than or equal to 1 μm.

Wax microdispersions are stable dispersions of colloidal wax particles, and are described, for example, in "Microemulsions Theory and Practice", L. M. Prince Ed., Academic Press (1977) pages 21-32.

For example, these wax microdispersions may be obtained by melting the wax in the presence of a surfactant, and optionally in the presence of a portion of water, followed by gradual addition of hot water with stirring. The intermediate formation of an emulsion of the water-in-oil type is observed, followed by a phase inversion, with final production of a microemulsion of the oil-in-water type. On cooling, a stable microdispersion of solid wax colloidal particles is obtained.

The wax microdispersions may also be obtained by stirring the mixture of wax, surfactant and water using stirring tools such as ultrasound, high-pressure homogenizers or turbomixers.

The particles of the wax microdispersion have, for example, mean sizes of less than 1 μm (such as ranging from 0.02 μm to 0.99 μm) and, for example, less than or equal to 0.5 μm (such as ranging from 0.06 μm to 0.5 μm).

These particles consist essentially of a wax or a mixture of waxes. However, they may comprise a small proportion of oily and/or pasty fatty additives, a surfactant and/or a common liposoluble additive/active agent.

When the wax or the mixture of waxes is present in the cosmetic compositions as disclosed herein in the form of an aqueous dispersion of particles, the size of the particles, i.e., the mean "effective" volume diameter D[4,3] as defined above, may be, for example, less than or equal to 1 μm such as less than or equal to 0.75 μm.

The wax particles may have varied shapes. For example, they may be spherical.

<u>Fibers</u>

In some embodiments, the cosmetic composition of the invention may further comprise at least one fiber to allow an improvement in the lengthening effect. The fibers useful in the present invention may be chosen from rigid or non-rigid fibers and may be of natural or synthetic fibers. Natural fibers include, but are not limited to, cotton, silk, wool, and other keratin fibers. Synthetic fibers include, but are not limited to, polyester, rayon, nylon, and other polyamide fibers. In some embodiments, fibers may be made of non-rigid fibers such as polyamide (Nylon®) fibers, or rigid fibers such as polyimide-amide fibers, for instance, those sold under the trade name "Kermel" and "Kermel Tech" by Rhodia, or poly(p-phenyleneterephthalamide) (or aramid) fibers sold especially under the name Kevlar® by DuPont de Nemours.

The fibers may be present in the cosmetic compositions in an amount generally ranging from 0.01% to 10% by weight of the total weight of the composition, including all ranges and subranges therebetween.

<u>Fillers</u>

The cosmetic composition of the invention may also comprise a filler selected from those that are well known to a person skilled in the art and commonly used in cosmetic compositions. The fillers should be understood to mean lamellar or non-lamellar, inorganic or organic particles. Representative examples of these ingredients include mica, silica, kaolin, iron oxides, titanium dioxide, polyamide powders, polyamide powders, for instance Nylon® (Orgasol from Atochem), poly-alanine powders, polyethylene powders, tetrafluoroethylene polymer powders, for instance Teflon®, lauroyllysine, starch, boron nitride, hollow polymer microspheres such as those of polyvinylidene chloride/acrylonitrile, for instance Expancel® (Nobel Industrie), acrylic powders such as Polytrap® (Dow Corning), polymethyl methacrylate particles and silicone resin microbeads (for example, Tospearls® from Toshiba), precipitated calcium carbonate, magnesium carbonate, magnesium hydrocarbonate, hydroxyapatite, hollow silica microspheres (Silica Beads® from Maprecos), glass or ceramic microcapsules, metal soaps derived from organic carboxylic acids containing from 8 to 22 carbon atoms, preferably from 12 to 18 carbon atoms, for example, zinc stearate, magnesium stearate, lithium stearate, zinc laurate, or magnesium myristate.

The fillers, if present, are in amounts generally ranging from 0.1% to 25%, and preferably from 1% to 20% by weight of the total weight of the composition, including all ranges and subranges therebetween.

<u>Dyestuffs</u>

According to the present invention, the cosmetic composition may optionally comprise at least one dyestuff. Suitable dyestuffs include but are not limited to pulverulent dyestuff, liposoluble dyes, and water-soluble dyes. This dyestuff may be in the cosmetic composition in a concentration ranging from 0.01% to 30% by weight of the total weight of the composition, including all ranges and subranges therebetween.

The pulverulent dyestuffs may be chosen from pigments and nacres.

The pigments, which may be used according to the present invention, may be chosen from white, colored, inorganic, organic, polymeric, nonpolymeric, coated and uncoated pigments. Representative examples of inorganic pigments include titanium dioxide, optionally surface-treated, zirconium oxide, zinc oxide, cerium oxide, iron oxide, chromium oxide, manganese violet, ultramarine blue, chromium hydrate, and ferric blue. Representative examples of

organic pigments include carbon black, pigments of D&C type, and lakes based on cochineal carmine, barium, strontium, calcium, and aluminum.

The nacres which may be used according to the present invention may be chosen from white nacreous pigments such as mica coated with titanium or with bismuth oxychloride, colored nacreous pigments such as titanium mica with iron oxides, titanium mica with iron oxides, titanium mica with ferric blue or chromium oxide, titanium mica with an organic pigment chosen from those mentioned above, and nacreous pigments based on bismuth oxychloride.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow.

The water-soluble dyes which may be used according to the present invention include beetroot juice, methylene blue, the disodium salt of ponceau, the disodium salt of alizarin green, quinoline yellow, the trisodium salt of amaranth, the disodium salt of tartrazine, the monosodium salt of rhodamine, the disodium salt of fuchsin, and xanthophyll.

Additives

The cosmetic composition of the invention can also comprise any additive usually used in the field under consideration. For example, dispersants such as poly(12-hydroxystearic acid), antioxidants, essential oils, sunscreens, preserving agents, fragrances, neutralizing agents, cosmetic and dermatological active agents such as, for example, emollients, moisturizers, vitamins, essential fatty acids, surfactants, pasty compounds, and mixtures thereof can be added. A non-exhaustive listing of such ingredients can be found in U.S. patent application publication no. 2004/0170586, the entire contents of which are hereby incorporated by reference. Still further examples of such additional ingredients may be found in the International Cosmetic Ingredient Dictionary and Handbook (9th edition, 2002).

A person skilled in the art will take care to select the optional additional additives and/or the amount thereof such that the advantageous properties of the cosmetic composition according to the invention are not, or are not substantially, adversely affected by the envisaged addition.

These substances may be selected variously by the person skilled in the art in order to prepare a composition which has desired properties, for example, consistency or texture.

These additives may be present in the cosmetic composition in a proportion from 0% to 99%, preferably from 0.01% to 90%, and more preferably from 0.1% to 50% by weight of the total weight of the composition, including all ranges and subranges therebetween.

Needless to say, the cosmetic composition of the invention should be cosmetically or dermatologically acceptable, i.e. it should contain a non-toxic physiologically acceptable medium and should be able to be applied to the eyelashes of human beings.

Cosmetic process

According to one embodiment of the present invention, a cosmetic process for making-up eyelashes is provided. This process comprises the steps of loading an applicator with the cosmetic composition of the present invention, and applying said cosmetic composition onto the eyelashes.

The cosmetic composition according to the invention is preferably a mascara.

The cosmetic composition according to the invention may be packed in a cosmetic product comprising a container delimiting at least one compartment which comprises the cosmetic composition, the container being closed by a closing member.

The container is preferably combined with an applicator, especially in the form of a brush comprising an arrangement of bristles maintained by a twisted wire. Such a twisted brush is described especially in patent US 4 887 622. It may also be in the form of a comb comprising a plurality of application members, obtained especially by moulding. Such combs are described for example in patent FR 2 796 529. The applicator may be integrally attached to the container, as described for example in patent FR 2 761 959. Advantageously, the applicator is integrally attached to a rod which is itself integrally attached to the closing member.

The closing member may be coupled to the container by screwing. Alternatively, the coupling between the closing member and the container is achieved by a method other than by screwing, especially via a bayonet mechanism, by click-fastening or by tightening. The term "click-fastening" in particular means any system involving the crossing of a bead or cord of material by elastic deformation of a porting, especially a closing member, followed by return to the elastically unconstrained position of the said portion after crossing of the bead or cord.

The container may be at least partially made of thermoplastic material. Examples of thermoplastic materials that may be mentioned include polypropylene or polyethylene.

Alternatively, the container is made of non thermoplastic material, especially glass or metal (or alloy).

The container is preferably equipped with a drainer arranged in the region of the aperture of the container.

Such a drainer makes it possible to wipe the applicator and possibly the rod to which it may be integrally attached. Such a drainer is described for example in patent FR 2 792 618.

EXAMPLES

The following examples are intended to further illustrate the present invention. They are not intended to limit the invention in any way. Unless otherwise indicated, the amounts indicated are weight percentages and are expressed relative to the total weight of the composition.

**Example 1 and Comparative Examples 1 to 4**

The cosmetic compositions (mascaras) for eyelashes according to Example 1 and Comparative Examples 1 to 4 which have the following formulas shown in Table 1 were prepared. The numerals in Table 1 are based on percentages by weight relative to the total weight of the composition.

For each of Example 1 and Comparative Examples 1 to 4, the powder components shown in Table 1 were mixed for about 10 minutes. The non-powder ingredients (oil, surfactant, and the like) shown in Table 1 were then added to the mixture, and mixed together for about 15 minutes to obtain the cosmetic composition for eyelashes.

Table 1

| **Ingredients** | **Ex. 1** | **Comp. Ex. 1** | **Comp. Ex. 2** | **Comp. Ex. 3** | **Comp. Ex. 4** |
|---|---|---|---|---|---|
| Copernicia Cerifera (carnauba) wax | 3.00 | 3.00 | 3.000 | 3.00 | 3.00 |
| Beeswax | 7.00 | 7.00 | 7.000 | 7.00 | 7.00 |
| | | | | | |
| Sorbitan stearate | 2.50 | 2.50 | 2.500 | 2.50 | 2.50 |
| 2-Amino-2-methyl-1,3-propanediol (AMPD) | 0.70 | 0.70 | 0.028 | - | - |
| Triethanolamine (TEA) | - | - | - | 0.70 | - |
| 2-Amino-2-methyl-1-propanol (AMP) | - | - | - | - | 0.70 |
| Stearic acid | 1.10 | 1.10 | 0.044 | 1.10 | 1.10 |
| Steareth-20 | 2.00 | 2.00 | 2.000 | 2.00 | 2.00 |
| Iron oxide (Black) | 10.00 | 10.00 | 10.000 | 10.00 | 10.00 |
| | | | | | |
| Water | 30.60 | 56.60 | 32.328 | 30.60 | 30.60 |
| Sodium EDTA | 0.10 | 0.10 | 0.100 | 0.10 | 0.10 |
| Hydroxyethylcellulose | 1.00 | 1.00 | 1.000 | 1.00 | 1.00 |
| Polyvinyl pyrrolidone (K30) | 2.00 | 2.00 | 2.000 | 2.00 | 2.00 |
| | | | | | |
| Sodium dehydroacetate | 0.20 | 0.20 | 0.200 | 0.20 | 0.20 |
| Phenoxyethanol | 0.50 | 0.50 | 0.500 | 0.50 | 0.50 |
| Ethylparaben | 0.10 | 0.10 | 0.100 | 0.10 | 0.10 |
| Methylparaben | 0.20 | 0.20 | 0.200 | 0.20 | 0.20 |
| Propylene glycol | 2.00 | 2.00 | 2.000 | 2.00 | 2.00 |
| STYRENE/ACRYLATES/AMMONIUM METHACRYLATE COPOLYMER AM(Active Material): 40% | 35.00 | 9.00 | 35.000 | 35.00 | 35.00 |
| Nylon-6 2mm fiber | 2.00 | 2.00 | 2.000 | 2.00 | 2.00 |

(continued)

| Ingredients | Ex. 1 | Comp. Ex. 1 | Comp. Ex. 2 | Comp. Ex. 3 | Comp. Ex. 4 |
|---|---|---|---|---|---|
| R | 0.13 | 0.50 | 0.005 | 0.13 | 0.13 |
| Total(w/w%) | 100.00 | 100.00 | 100.00 0 | 100.00 | 100.00 |
| Note: "R" = the weight ratio of the amount of AMPD, TEA or AMP in combination with the $C_{16}$-$C_{24}$ fatty acid / the amount of the film forming polymer | | | | | |

[Removability Test]

0.008 grams of the cosmetic composition of each of Example 1 and Comparative Examples 1 to 4 was applied to a fake lash ("Eprouvette Avec Segment de Cil 30 Noeuds Support 30x30mm PQ de 486" obtained from SP Equation) using a mascara brush (60 strokes). The fake lash was dried naturally at room temperature for 2 hours. Then, the fake lash was placed in warm water at 42°C for 1 minute. Immediately after taking out the fake lash from the warm water, the fake lash was pinched with a sheet ("Bioskin A4 5T #10" obtained from Beaulax) of 5cm*5cm square, and the cosmetic composition on the fake lash was removed by moving the bio skin toward the edge of the fake lash. Then, the sheet was unfolded to observe the depositions (the cosmetic composition and water) on the surface of the sheet. The results are shown in Table 2.

Table 2

| | Conditions of the removed cosmetic composition | Conditions of water |
|---|---|---|
| Ex. 1 | Tube (removed as a "tube") | clean (no smudge) |
| Comp. Ex. 1 | Liquid (removed as a "liquid") | smudged |
| Comp. Ex. 2 | No or slight residue (hard to remove) | clean (no smudge) |
| Comp. Ex. 3 | Mixture of tube and liquid | slightly smudged |
| Comp. Ex. 4 | Mixture of tube and liquid | slightly smudged |

As shown in Table 2, the cosmetic composition according to Example 1 exhibited good makeup removal properties. In other words, the mascara of Example 1 was easily removed as a tube without using any cleansing agents. Moreover, no smudge was observed in the removability test for Example 1.

In contrast, the cosmetic composition according to Comparative Example 1 with a higher weight ratio of "R" was removed as a liquid, and water smudge was observed. Thus, the mascara of Comparative Example 1 leads to a smudge issue on the consumer's face after cleansing with warm water.

The cosmetic composition according to Comparative Example 2 with a lower weight ratio of "R" was very difficult to remove with warm water.

The cosmetic compositions according to Comparative Examples 3 and 4, which comprise triethanolamine (TEA) and 2-amino-2-methyl-1-propanol (AMP) respectively instead of 2-amino-2-methyl-1,3-propanediol as in Example 1, were removed as a mixture of a tube and a liquid, and slight smudges in warm water were observed. Thus, the mascaras of Comparative Examples 3 and 4 could also lead to a smudge issue on the consumer's face after cleansing with warm water.

[Model Evaluation Test]

The cosmetic composition of each of Example 1 and Comparative Examples 1 and 2 were subjected to sensory evaluation tests with regard to some cosmetic properties by 8 testers under the following criteria shown in Table 3. Each property was evaluated in 10 score stages, i.e., from scores 1 to 10.

Table 3

| Scores | Criteria |
|---|---|
| 1 to 2 | Very poor and highly dissatisfied |

(continued)

| Scores | Criteria |
|---|---|
| 3 to 4 | Poor and dissatisfied |
| 5 to 6 | Fair |
| 7 to 8 | Good and satisfied |
| 9 to 10 | Very good and very satisfied |

The average of the scores for each sample was sorted in accordance with the following standard. The results are shown in Table 4.

○○○: 10.0 to 8.0
○○: less than 8.0 to 6.0
○: less than 6.0 to 4.0
▲ : less than 4.0 to 2.0
x: less than 2.0

Table 4

| | Ex. 1 | Comp. Ex. 1 | Comp. Ex. 2 |
|---|---|---|---|
| Smooth brush application | ○○○ | ○○○ | ▲ |
| Deposit on eyelashes | ○○ | ○○ | ▲ |
| Curling effect | ○○○ | ○ | ○○ |
| Lengthening effect | ○○○ | ○○ | ○ |
| Lasting effect | ○○ | ▲ | ○○○ |
| Regularity and Fringe property after application | ○○○ | ○○ | ○○ |
| Tube removability with warm water | ○○○ | ▲ | ○ |
| Clean makeup removability (No-Panda eyes) after warm water cleansing | ○○○ | ▲ | ○○○ |

As shown in Table 4, the cosmetic composition according to Example 1 exhibited not only better tube removability with warm water and clean makeup removability after warm water cleansing but also better make-up properties such as good curling effects and good lengthening effects than those according to Comparative Examples 1 and 2.

## Claims

**1.** A cosmetic composition for eyelashes, comprising:

- at least one anionic surfactant including 2-amino-2-methyl-1,3-propanediol in combination with a $C_{16}$-$C_{24}$ fatty acid; and
- at least one film-forming polymer present in the form of particles dispersed in an aqueous phase,

wherein
the weight ratio of the amount of the 2-amino-2-methyl-1,3-propanediol in combination with the $C_{16}$-$C_{24}$ fatty acid/the amount of the film forming polymer is 0.01 to 0.3, preferably 0.05 to 0.2, and more preferably 0.1 to 0.15, and
the film-forming polymer is selected from vinyl (co)polymers, (meth)acrylic (co)polymers, urethanes (co)polymers, and mixtures thereof.

**2.** The cosmetic composition for eyelashes according to Claim 1, wherein the $C_{16}$-$C_{24}$ fatty acid is stearic acid.

**3.** The cosmetic composition for eyelashes according to Claim 1 or 2, wherein the anionic surfactant is present in an

amount of from 0.01% to 10% by weight, preferably 0.1% to 5% by weight, and more preferably 0.5 to 3% by weight, relative to the total weight of the cosmetic composition.

**4.** The cosmetic composition for eyelashes according to any one of Claims 1 to 3, wherein the film-forming polymer is selected from styrene-(meth)acrylic and (meth)acrylic copolymer, vinyl acetate and (meth)acrylic copolymer and mixtures thereof.

**5.** The cosmetic composition for eyelashes according to any one of Claims 1 to 4, wherein the film-forming polymer is present in an amount of dry matter from 10% to 30% by weight, preferably from 15% to 25% by weight, and more preferably 15% to 20% by weight, relative to the total weight of the cosmetic composition.

**6.** The cosmetic composition for eyelashes according to any one of Claims 1 to 5, further comprising at least one nonionic surfactant with an HLB of less than 8 at 25°C.

**7.** The cosmetic composition for eyelashes according to Claim 6, wherein the nonionic surfactant with an HLB of less than 8 at 25°C is selected from the group consisting of saccharide esters and ethers, fatty acid esters, a mixture of cyclomethicone/dimethicone copolyol, and mixtures thereof.

**8.** The cosmetic composition for eyelashes according to any one of Claims 1 to 7, further comprising at least one nonionic surfactant with an HLB of greater than or equal to 8 at 25°C.

**9.** The cosmetic composition for eyelashes according to Claim 8, wherein the nonionic surfactant with an HLB of greater than or equal to 8 at 25°C is selected from the group consisting of oxyethylenated and/or oxypropylenated ethers of glycerol, oxyethylenated and/or oxypropylenated ethers of fatty alcohol, fatty acid esters of polyethylene glycol, fatty acid esters of oxyethylenated and/or oxypropylenated glyceryl ethers, fatty acid esters of oxyethylenated and/or oxypropylenated sorbitol ethers, dimethicone copolyol, dimethicone copolyol benzoate, copolymers of propylene oxide and of ethylene oxide, and mixtures thereof.

**10.** The cosmetic composition for eyelashes according to any one of Claims 1 to 9, comprising water in an amount ranging from 15% to 50% by weight, preferably 20% to 40% by weight, and more preferably 25% to 35% by weight, relative to the total weight of the cosmetic composition.

**11.** The cosmetic composition for eyelashes according to any one of Claims 1 to 10, further comprising at least one wax.

**12.** The cosmetic composition for eyelashes according to any one of Claims 1 to 11, further comprising at least one fiber.

**13.** A cosmetic process for making-up eyelashes, comprising the steps of:

- loading an applicator with the cosmetic composition according to any one of Claims 1 to 12, and
- applying said cosmetic composition onto the eyelashes.

**Patentansprüche**

**1.** Kosmetische Zusammensetzung für Wimpern, umfassend:

- mindestens ein anionisches Tensid, das 2-Amino-2-methyl-1,3-propandiol in Kombination mit einer $C_{16}$-$C_{24}$-Fettsäure enthält; und
- mindestens ein filmbildendes Polymer, das in Form von in einer wässrigen Phase dispergierten Partikeln vorliegt,

wobei
das Gewichtsverhältnis der Menge des 2-Amino-2-methyl-1,3-propandiols in Kombination mit der $C_{16}$-$C_{24}$-Fettsäure zu der Menge des filmbildenden Polymers 0,01 bis 0,3, vorzugsweise 0,05 bis 0,2 und mehr bevorzugt 0,1 bis 0,15 beträgt, und
das filmbildende Polymer aus Vinyl(co)polymeren, (Meth)acrylsäure(co)polymeren, Urethan(co)polymeren und Mischungen davon ausgewählt ist.

2. Kosmetische Zusammensetzung für Wimpern nach Anspruch 1, wobei es sich bei der $C_{16}$-$C_{24}$-Fettsäure um Stearinsäure handelt.

3. Kosmetische Zusammensetzung für Wimpern nach Anspruch 1 oder 2, wobei das anionische Tensid in einer Menge von 0,01 bis 10 Gew.-%, vorzugsweise von 0,1 bis 5 Gew.-% und mehr bevorzugt von 0,5 bis 3 Gew.-% vorliegt, bezogen auf das Gesamtgewicht der kosmetischen Zusammensetzung.

4. Kosmetische Zusammensetzung für Wimpern nach einem der Ansprüche 1 bis 3, wobei das filmbildende Polymer aus Styrol-(Meth)acrylsäure und (Meth)acrylsäure-Copolymer, Vinylacetat und (Meth)acrylsäure-Copolymer sowie Mischungen davon ausgewählt ist.

5. Kosmetische Zusammensetzung für Wimpern nach einem der Ansprüche 1 bis 4, wobei das filmbildende Polymer in einer Menge an Trockenmasse von 10 bis 30 Gew.-%, vorzugsweise von 15 bis 25 Gew.-% und mehr bevorzugt von 15 bis 20 Gew.-% vorliegt, bezogen auf das Gesamtgewicht der kosmetischen Zusammensetzung.

6. Kosmetische Zusammensetzung für Wimpern nach einem der Ansprüche 1 bis 5, ferner umfassend mindestens ein nichtionisches Tensid mit einem HLB-Wert kleiner als 8 bei 25°C.

7. Kosmetische Zusammensetzung für Wimpern nach Anspruch 6, wobei das nichtionische Tensid mit einem HLB-Wert kleiner als 8 bei 25°C aus der aus Saccharidestern und -ethern, Fettsäureestern, einer Mischung von Cyclomethicon/Dimethicon-Copolyol sowie Mischungen davon bestehenden Gruppe ausgewählt ist.

8. Kosmetische Zusammensetzung für Wimpern nach einem der Ansprüche 1 bis 7, ferner umfassend mindestens ein nichtionisches Tensid mit einem HLB-Wert größer oder gleich 8 bei 25°C.

9. Kosmetische Zusammensetzung für Wimpern nach Anspruch 8, wobei das nichtionische Tensid mit einem HLB-Wert größer oder gleich 8 bei 25°C aus der aus oxyethylenierten und/oder oxypropylenierten Ethern von Glycerin, oxyethylenierten und/oder oxypropylenierten Ethern von Fettalkohol, Fettsäureestern von Polyethylenglycol, Fettsäureestern von oxyethylenierten und/oder oxypropylenierten Glycerylethern, Fettsäureestern von oxyethylenierten und/oder oxypropylenierten Sorbitethern, Dimethiconcopolyol, Dimethiconcopolyolbenzoat, Copolymeren von Propylenoxid und von Ethylenoxid sowie Mischungen davon bestehenden Gruppe ausgewählt ist.

10. Kosmetische Zusammensetzung für Wimpern nach einem der Ansprüche 1 bis 9, umfassend Wasser in einer Menge im Bereich von 15 bis 50 Gew.-%, vorzugsweise 20 bis 40 Gew.-% und mehr bevorzugt 25 bis 35 Gew.-%, bezogen auf das Gesamtgewicht der kosmetischen Zusammensetzung.

11. Kosmetische Zusammensetzung für Wimpern nach einem der Ansprüche 1 bis 10, ferner umfassend mindestens ein Wachs.

12. Kosmetische Zusammensetzung für Wimpern nach einem der Ansprüche 1 bis 11, ferner umfassend mindestens eine Faser.

13. Kosmetisches Verfahren zum Schminken von Wimpern, mit den folgenden Schritten:

- Füllen eines Applikators mit der kosmetischen Zusammensetzung nach einem der Ansprüche 1 bis 12, und
- Auftragen der kosmetischen Zusammensetzung auf die Wimpern.

## Revendications

1. Composition cosmétique pour cils, comprenant :

- au moins un tensioactif anionique comprenant du 2-amino-2-méthyl-propane-1,3-diol en combinaison avec un acide gras en $C_{16}$-$C_{24}$,
- et au moins un polymère filmogène, présent sous la forme de particules dispersées au sein d’une phase aqueuse,

dans laquelle

- le rapport en poids de la quantité de 2-amino-2-méthyl-propane-1,3-diol en combinaison avec l'acide gras en $C_{16}$-$C_{24}$ à la quantité de polymère filmogène vaut de 0,01 à 0,3, de préférence de 0,05 à 0,2 et mieux encore de 0,1 à 0,15,
- et le polymère filmogène est choisi parmi les polymères et copolymères polyvinyliques, les polymères et copolymères polyacryliques ou polyméthacryliques, les polymères et copolymères polyuréthanes, et les mélanges de tels polymères.

**2.** Composition cosmétique pour cils conforme à la revendication 1, dans laquelle l'acide gras en $C_{16}$-$C_{24}$ est de l'acide stéarique.

**3.** Composition cosmétique pour cils conforme à la revendication 1 ou 2, dans laquelle le tensioactif anionique se trouve présent en une proportion de 0,01 à 10 %, de préférence de 0,1 à 5 % et mieux encore de 0,5 à 3 %, en poids rapporté au poids total de la composition cosmétique.

**4.** Composition cosmétique pour cils conforme à l'une des revendications 1 à 3, dans laquelle le polymère filmogène est choisi parmi les copolymères de styrène et de monomère acrylique ou méthacrylique, les copolymères d'acétate de vinyle et de monomère acrylique ou méthacrylique, et les mélanges de tels copolymères.

**5.** Composition cosmétique pour cils conforme à l'une des revendications 1 à 4, dans laquelle le polymère filmogène se trouve présent en un poids de matière sèche représentant de 10 à 30 %, de préférence de 15 à 25 % et mieux encore de 15 à 20 % du poids total de la composition cosmétique.

**6.** Composition cosmétique pour cils conforme à l'une des revendications 1 à 5, qui comprend en outre au moins un tensioactif non-ionique présentant un rapport hydro-lipophile inférieur à 8 à 25 °C.

**7.** Composition cosmétique pour cils conforme à la revendication 6, dans laquelle le tensioactif non-ionique présentant un rapport hydro-lipophile inférieur à 8 à 25 °C est choisi dans l'ensemble formé par les esters et éthers de saccharide, les esters d'acide gras, un mélange de cyclométhicone et de diméthicone-copolyol, et les mélanges de tels composés.

**8.** Composition cosmétique pour cils conforme à l'une des revendications 1 à 7, qui comprend en outre au moins un tensioactif non-ionique présentant un rapport hydro-lipophile supérieur ou égal à 8 à 25 °C.

**9.** Composition cosmétique pour cils conforme à la revendication 8, dans laquelle le tensioactif non-ionique présentant un rapport hydro-lipophile supérieur ou égal à 8 à 25 °C est choisi dans l'ensemble formé par les éthers éthoxylés et/ou propoxylés de glycérol, les éthers éthoxylés et/ou propoxylés d'alcool gras, les esters d'acide gras et de polyéthylèneglycol, les esters d'acide gras et d'éther éthoxylé et/ou propoxylé de glycérol, les esters d'acide gras et d'éther éthoxylé et/ou propoxylé de sorbitol, le diméthicone-copolyol, le benzoate de diméthicone-copolyol, les copolymères d'oxyde de propylène et d'oxyde d'éthylène, et les mélanges de tels composés.

**10.** Composition cosmétique pour cils conforme à l'une des revendications 1 à 9, qui comprend de l'eau en une proportion de 15 à 50 %, de préférence de 20 à 40 % et mieux encore de 25 à 35 %, en poids rapporté au poids total de la composition cosmétique.

**11.** Composition cosmétique pour cils conforme à l'une des revendications 1 à 10, qui comprend en outre au moins une cire.

**12.** Composition cosmétique pour cils conforme à l'une des revendications 1 à 11, qui comprend en outre des fibres d'au moins une sorte.

**13.** Procédé cosmétique de maquillage de cils, comportant les étapes suivantes :

- mettre dans un applicateur une composition cosmétique conforme à l'une des revendications 1 à 12,
- et appliquer ladite composition cosmétique sur des cils.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008062530 A
- FR 2232303 A
- EP 847752 A
- FR 2792190 A
- US 20040170586 A
- US 4887622 A
- FR 2796529
- FR 2761959
- FR 2792618


### Non-patent literature cited in the description


- **VAN DE HULST, H. C.** Light Scattering by Small Particles. Wiley, 1957
- Microemulsions Theory and Practice. Academic Press, 1977, 21-32
- International Cosmetic Ingredient Dictionary and Handbook. 2002